Europäisches Patentamt

European Patent Office

Office européen des brevets

(19)

(11) Publication number: **0 230 786 B1**

# EUROPEAN PATENT SPECIFICATION

(12)

(45) Date of publication of patent specification: **25.09.91**

(51) Int. Cl.⁵: **C12M 1/40**, C12Q 1/60, G01N 33/48

(21) Application number: **86310151.5**

(22) Date of filing: **24.12.86**

(54) **Assay for cholesterol and derivatives thereof.**

(30) Priority: **24.12.85 GB 8531755**

(43) Date of publication of application:
**05.08.87 Bulletin 87/32**

(45) Publication of the grant of the patent:
**25.09.91 Bulletin 91/39**

(84) Designated Contracting States:
**AT BE CH DE ES FR GB GR IT LI LU NL SE**

(56) References cited:
**EP-A- 0 121 385**
**EP-A- 0 125 867**

**CHEMICAL ABSTRACTS, vol. 101, no. 5, 30th July 1984, page 242, abstract no. 35287e, Columbus, Ohio, US; U. WOLLENBERGER et al.: "Amperometric enzyme sequence electrodes for cholesterol", & BIOELECTROCHEM. BIOENERG. 1983, 11(4-6), 307-317**

**CHEMICAL ABSTRACTS, vol. 103, no. 13, 30th September 1985, page 312, abstract no. 101465f, Columbus, Ohio, US; T. YAO et al.: "Amperometric assays of total and free cholesterols in serum by the combined use of immobilized cholesterol esterase and cholesterol oxidase reactors and peroxidase electrode in a flow injection system", & ANAL. BIOCHEM. 1985, 149(2), 387-391**

(73) Proprietor: **MediSense, Inc.**
**128 Sidney Street**
**Cambridge Massachusetts 02139(US)**

(72) Inventor: **Hill, Hugh Allen Oliver**
**38 Nuffield Way**
**Abingdon Oxon OX14 1RL(GB)**
Inventor: **Frew, Jane Elizabeth**
**38 Nuffield Way**
**Abingdon Oxon OX14 1RL(GB)**
Inventor: **Ball, Michael Richard**
**38 Nuffield Way**
**Abingdon Oxon OX14 1RL(GB)**
Inventor: **Green, Monika Joanna**
**38 Nuffield Way**
**Abingdon Oxon OX14 1RL(GB)**

(74) Representative: **Ruffles, Graham Keith et al**
**MARKS & CLERK 57-60 Lincoln's Inn Fields**
**London WC2A 3LS(GB)**

**Description**

The present invention relates to an assay for free or total cholesterol in blood.

BACKGROUND OF THE INVENTION

Elevated levels of cholesterol and its esters are recognised as risk factors for arteriosclerosis and myocardial infarction. The normal range of serum values extends from 3 to 6 mM for total cholesterol (free cholesterol and cholesterol ester), while in hyperlipidaemic conditions the level can increase to I0 mM and above.

Procedures for cholesterol determination have employed a variety of chemical and physical techniques. Traditional assays involve chemical treatment of cholesterol to yield coloured products which can be measured spectrophotometrically. The most common reactions are the Liebermann-Burchard reaction, the iron salt-sulphuric acid reaction, and the p-toluene sulphonic acid reaction. However, these various traditional procedures involve numerous drawbacks, including a lack of specificity, difficulty in standardization, the use of unstable and corrosive reagents, a variable reactivity of the esters, and interference by other molecules such as haemoglobin and bilirubin.

Most modern methods for blood cholesterol determination are enzymic, and take advantage of the specificity afforded by enzymes. Typically the detection is spectrophotometric. A chromogen is generated through a series of stoichiometrically coupled reactions, such as;

(A): cholesterol ester + $H_2O$ = cholesterol + fatty acid

(B): cholesterol + $O_2$ = cholestenone + $H_2O_2$

(C): $H_2O_2$ + phenol + 4-aminophenazone = 4-(p-benzoquinone-monoimino)-phenazone + $4H_2O$

The reactions (A), (B), and (C) are respectively catalysed by a cholesterol esterase, a cholesterol oxidase, and a peroxidase.

The phenazone derivative formed in reaction (C) has specific spectroscopic properties and therefore the cholesterol concentration in a sample can be determined by the amount of the phenazone derivative produced under appropriate conditions.

The disadvantages of such methods include the particular difficulties of calibration when blood plasma is being assayed, especially where the turbidity of the plasma must be taken into consideration. Moreover, this method is not suitable for assay of cholesterol in whole blood and consequently an expensive and time-consuming preparation of the sample must be carried out.

Other techniques may be employed for determination of the products of enzymic assays for cholesterol. For example, European Patent Application 84303085, published as EPI25867, describes a method of assay in which an electrode poised at a suitable potential is contacted with a system comprising a first enzyme, a cofactor linked with said enzyme and a mediator compound which transfers charge to the electrode from the first enzyme when its electrical state is changed by reaction of cofactor material. The cofactor may be $NAD^+$, $NADP^+$, cAMP, ATP, GTP, TTP, or CTP.

In one example of a procedure in accordance with EPI25867, an electrochemical determination of cholesterol is performed by oxidizing cholesterol to cholestenone using cholesterol dehydrogenase in the presence of $NAD^+$ as cofactor, followed by oxidation of the resultant NADH with a diaphorase whose catalytic activity is detected at an electrode using a ferrocene compound as a mediator.

In practice, there are some difficulties in adapting such a detection procedure for use with blood samples. Specifically, it seems that the enzymes are extremely sensitive to inhibition by surfactants of the kind conventionally employed to release cholesterol esters from the lipoprotein complex in whole sera.

Amperometric assays in a flow injection system and in a conventional measuring cell are disclosed respectively in Anal. Biochem. (1985) 149, 387-391 and Bioelectrochem. Bioenerg. (1983) 11, 307-307, and may thus be described as wet systems.

OBJECTS OF THE INVENTION

It is an object of this invention to provide an assay which can be performed rapidly, accurately and at low cost by persons such as general medical practioners in their own surgery. It is a further object of the present invention to provide sensors for such an assay. A specific object of this invention is an assay which can be performed without the need for elaborate preliminary treatment of a blood sample.

SUMMARY OF THE INVENTION

The present invention provides a sensor for use in the assay of cholesterol in blood. The sensor is a dry strip sensor for connection to readout means, and comprises an elongate electrically-insulating substrate with a screen printed reference electrode and a screen printed working electrode. The working electrode comprises a conducting surface, preferably of finely divided carbon optionally with a silver tracking, carrying a reagent mix. The reagent mix includes a surfactant, a cholesterol oxidase, a peroxidase, and a mediator which transfers electrons between the conducting surface and the peroxidase in the presence of peroxide as substrate. The mix further includes a cholesterol esterase if assay of total cholesterol is desired.

Without being bound by theory, it is believed that in an assay in accordance with the present invention, the surfactant serves to break up the lipoprotein complex of blood, and cholesterol is then oxidised to cholestenone by the cholesterol oxidase. Such oxidation is accompanied by oxygen to hydrogen peroxide which is then itself further reduced to water by the peroxidase. The catalytic activity of the peroxidase is detected at an electrode using the mediator to transfer electrons. If the optional cholesterol esterase is present, then cholesterol esters are first converted to cholesterol, which will not occur if the assay is only for free cholesterol and the esterase is omitted.

It seems that in the present assay, the mediator is electrochemically reduced at the electrode, producing a current measurable at the electrode, which current is related to the activity of the cholesterol oxidase and hence the amount of cholesterol present in the sample.

## FEATURES OF THE INVENTION

It is a feature of this invention that the sensor is a dry strip sensor. Surprisingly, it was found that a similar mix of reagents employed in a wet sensor system did not give good results across a desired range of detectable cholesterol concentrations.

It is a further feature of this invention that the dry strip sensor is produced with the aid of screen printing. Given that the reagent mix includes enzymes, it will be implicit that a low temperature printing procedure is required.

The mediator compound may be selected from ferrocyanide. ruthenium compounds, carboranes, polyviologens, one-dimensional conductive salts of TCNQ, haloanils and derivatives thereof, alkyl substituted phenazine derivatives, and, bis-cyclopentadienyl $(Cp)_2 MX_x$ complexes including ferrocene and its derivatives. The mediator is preferably ferrocene or a derivative thereof, especially a water-soluble ferrocene derivative.

In a particularly preferred embodiment, the dry strip sensor of this invention comprises an elongate, electrically-insulating substrate having a pair of longitudinal, substantially parallel, electrically-conducting tracks thereupon, each track being provided at the same end thereof with means for electrical connection with read-out means and provided at the other end thereof with an electrode, one of the electrodes being the reference electrode and the other being the working electrode responsive to the presence of cholesterol or its esters.

In performing an assay, the working electrode is preferably fixed at a small negative voltage relative to an Ag/AgCl reference counter-electrode.

The present invention will be further illustrated by way of example and with reference to the accompanying drawings.

## SUMMARY OF THE DRAWINGS

In the drawings,
Figure I shows a screen-printed sensor according to the present invention,
Figure 2 shows a graph of hydrogen peroxide concentration against current in $\mu A$, for a sensor according to the present invention,
Figure 3 shows a graph of cholesterol concentration against current in $\mu A$, for a sensor according to the present invention,
Figure 4 shows a graph of cholesterol ester concentration against current in $\mu A$, for a sensor according to the present invention, and
Figures 5 and 6 show comparative results obtained with wet systems not in accordance with the present invention.

## EXAMPLES OF THE INVENTION

Example I: construction of dry strip sensor

Commercially obtained horse radish peroxidase, cholesterol oxidase, and cholesterol esterase were reconstituted in an imidazole buffer mix of pH 7 for 4 hours prior to the addition of finely divided carbon. The working electrode was then screen-printed onto a preprinted carbon track laid down upon a PVC substrate. The mix was allowed to dry.

The screen-printing process may comprise the following steps;
a) printing of a conductive tracking,
b) printing of a working electrode,
c) printing of a reference electrode, and,
d) printing of a dielectric insulation.

Figure I shows a PVC sheet (I) which comprises the supporting substrate for the electrode. Conductive carbon tracking (2) is screen-printed onto the surface of the substrate in two conductive tracks of 45mm by 2mm, with a separation of 3mm. These tracks are overlaid with silver conductive ink to form contacts (3) for connection of the sensor to readout apparatus.

The working electrode (5) and the reference counter-electrode (6) are applied to the ends of the tracking (2) by screen printing. The Ag/AgCl reference electrode (6) employed in the present instance is a quasi-reference electrode which adopts a potential determined by a redox couple added to the reference electrode in the dry phase.

If desired, a mesh can be applied over the printed electrodes to give protection. The mesh can be coated with one or more of the electrode reagents.

In the present example, the tracks (2) and the conductive silver ink do not extend the complete length of the substrate (I), facilitating manipulation of the sensor and insertion into readout apparatus.

Example 2; calibration of electrodes.

A sample of the species to be determined in buffer, blood or serum is then applied to a target area covering both the working electrode and the reference counter-electrode. Calibration curves were calculated for the three analytes, cholesterol ester, free cholesterol or $H_2O_2$.

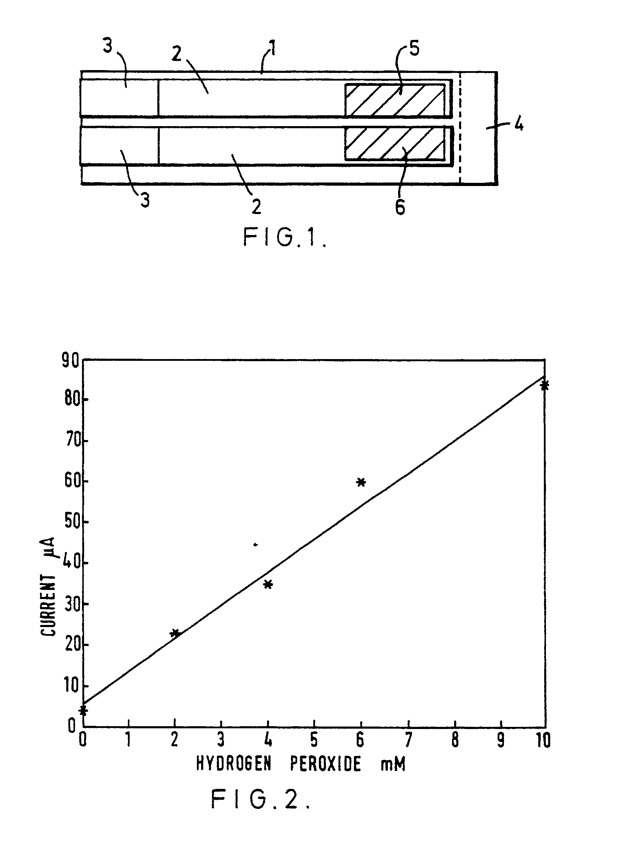

During calibration the potential was poised at -50mV. Fixed potential studies employing a range of hydrogen peroxide concentrations (0-I0mM) in imidazole (pH 7.0) buffer and in serum were carried out. A linear steady-state current response with respect to hydrogen peroxide concentration was recorded between 0.ImM and I0mM hydrogen peroxide. The results of one such calibration experiment are shown in figure 2, in which hydrogen peroxide concentration is plotted against current in $\mu$A, for a sensor according to the present invention.

Example 3: cholesterol detection

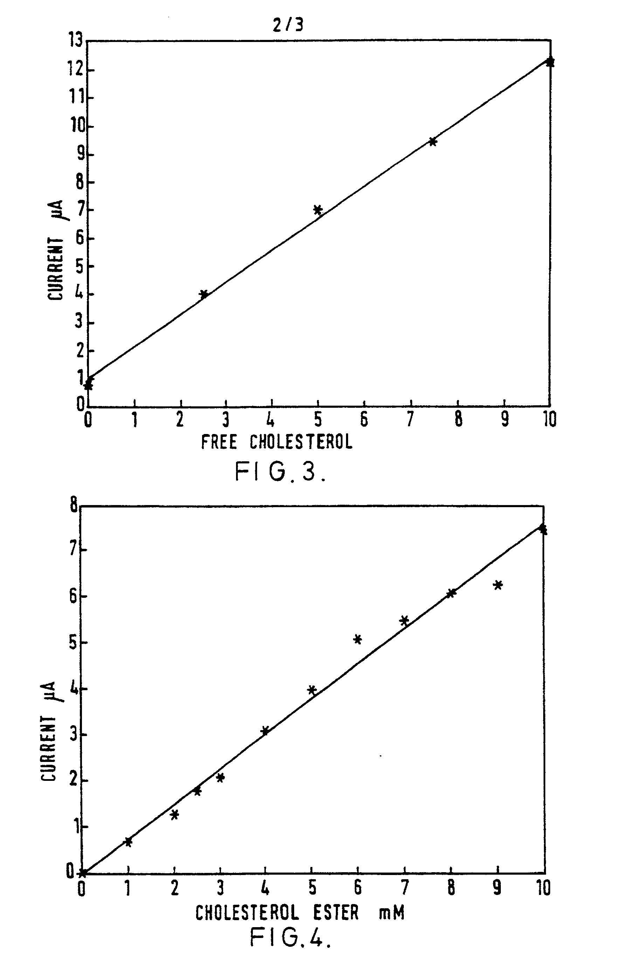

A study similar to that described in Example 2 was carried out using cholesterol standards (Sigma Chemical Company). Steady state currents proportional to the cholesterol concentration were obtained giving a linear relationship from ImM to I0mM. The results of one such calibration experiment are shown in figure 3, in which cholesterol concentration is plotted against current in $\mu$A, for a sensor according to the present invention.

Example 4: cholesterol ester detection.

A range of samples with varying cholesterol ester concentrations was made up using a formulation containing a surfactant. The nature of the surfactant is not critical, and suitable trial will establish if a candidate surfactant gives adequate breakdown of the blood lipoprotein complex. It was found possible to make up I0mM cholesterol ester in I0% surfactant.

The samples were applied to the electrode target area and allowed to incubate for four minutes at a poised potential of -50mV. Current values proportional to the cholesterol ester concentrations were obtained. The results of such an experiment are shown in figure 4, in which cholesterol ester concentration is plotted against current in $\mu$A, for a sensor according to the present invention.

Comparative Example: a wet system

An electrochemical glass cell with a working volume of I ml was set up with a gold working electrode and a platinum gauze as counter electrode. A mix of assay reagents was made up to 0.7 ml, and transferred to the cell. After appropriate poising of the working electrode, the current response was monitored on additon of the enzyme.

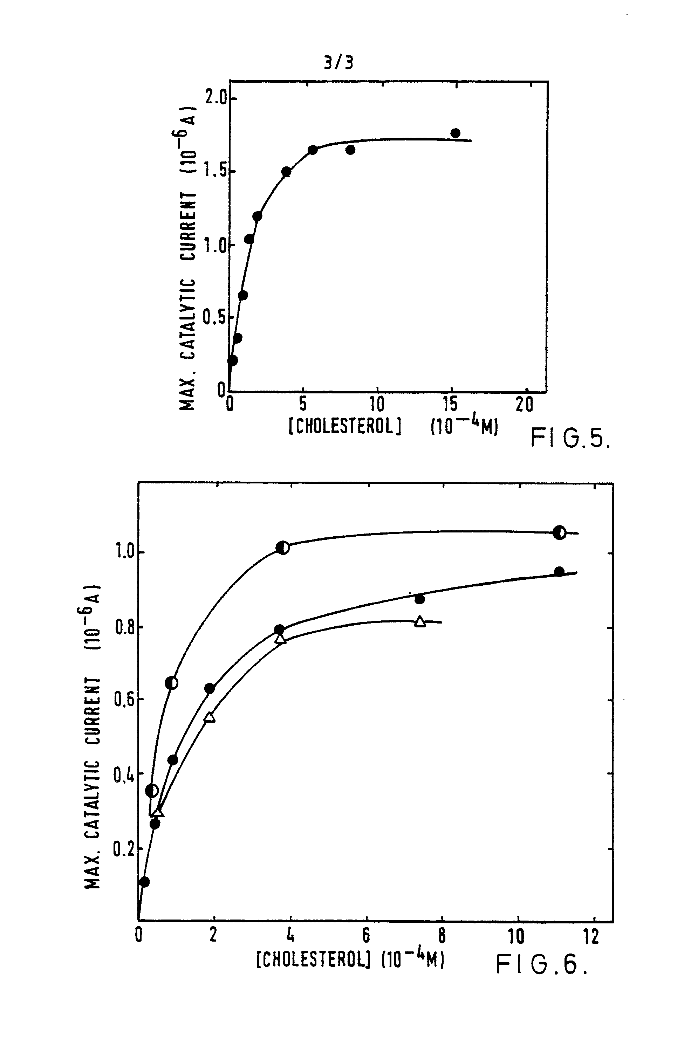

In a first experiment, for which the results are shown in Figure 5, the mediator was ferrocene methylamine hydrochloride and the working electrode potential was +I05 mV. For a second experiment, Figure 6, ferrocene monocarboxylic acid was employed with a working electrode potential of +44 mV and varying concentrations of cholesterol oxidase.

It will be seen that a non-linear response was obtained for each of these wet systems. In further experiments, it was established that the use of the dry strip sensor was essential to the present invention.

## Claims

1. A sensor for use in the assay of free or total cholesterol in blood, the sensor being a dry strip sensor for connection to read-out means and comprising an elongate electrically-insulating substrate with a screen printed reference electrode and a screen printed working electrode, the working electrode comprising a conducting surface carrying a surfactant, a cholesterol oxidase, a peroxidase, and a mediator which transfers electrons between the conducting surface and the peroxidase in the presence of peroxide as substrate, and further carrying cholesterol esterase in the case of an assay for total cholesterol.

2. The sensor of claim 1, which comprises the elongate, electrically-insulating substrate having a pair of longitudinal, substantially parallel, electrically-conducting tracks thereupon, each track being provided at the same end thereof with means for electrical connection with the read-out means and provided at the other end thereof with an electrode, one of the electrodes being the reference electrode and the other being the working electrode responsive to the presence of cholesterol.

3. An assay for free or total cholesterol, wherein the cholesterol is amperometrically quantitated using a sensor as defined in claim 1.

**Revendications**

1. Un capteur Pour utilisation lors du dosage du cholestérol libre ou total dans le sang, le capteur étant un capteur sec sur languette, pouvant être connecté à un moyen d'affichage et comprenant un substrat allongé à isolation électrique, avec une électrode de référence imprimée par sérigraphie et une électrode de travail imprimée par sérigraphie, l'électrode de travail comprenant une surface conductrice portant un agent tensio-actif, une cholestérol-oxydase, une peroxydase et un médiateur transférant des électrons entre la surface conductrice et la peroxydase en présence de peroxyde comme substrat, et portant en outre la cholestérol-estérase, en cas d'un dosage du cholestérol total.

2. Le capteur selon la revendication 1, comprenant le substrat allongé, à isolation électrique, le substrat comportant une paire de pistes longitudinales, pratiquement parallèles, électroconductrices, chaque piste étant pourvue à la même extrémité du substrat d'un moyen pour la connexion électrique avec le moyen d'affichage et à l'autre extrémité du substrat d'une électrode, l'une des électrodes faisant fonction d'électrode de référence et l'autre électrode faisant fonction d'électrode de travail, réagissant à la présence de cholestérol.

3. Un dosage du cholestérol libre ou total, le cholestérol étant dosé par ampérométrie avec utilisation d'un capteur comme défini dans la revendication 1.

**Patentansprüche**

1. Sensor zur Verwendung bei der Bestimmung von freiem oder Gesamtcholesterin im Blut, wobei der Sensor ein Trockenstreifen-Sensor für einen Anschluß an ein Ablesegerät ist und ein längliches, dielektrisches Subbstrat mit einer Siebdruck-Bezugselektrode und einer siebdruck-Arbeitselektrode umfaßt, wobei die Arbeitselektrode eine leitende Oberfläche umfaßt, auf der eine oberflächenaktive Substanz, eine Cholesterin-Oxydase, eine Peroxydase und eine Überträgersubstanz, die die Elektronen zwischen der leitenden Oberfläche und der Peroxydase bei Vorhandensein von Peroxydase als Substrat überträgt, aufgebracht sind, und auf der des weiteren bei einer Bestimmung von Gesamtcholesterin Cholesterin-Esterase aufgebracht ist.

2. Sensor nach Anspruch 1, der das längliche, dielektrische Substrat, auf dem sich zwei im wesentlichen parallele, elektrisch-leitende Längsspuren befinden, umfaßt, wobei jede Spur am gleichen Ende mit einem Mittel für die elektrische Verbindung mit dem Ablesegerät und am anderen Ende mit einer Elektrode versehen ist, wobei eine der Elektroden die Bezugselektrode und die andere die Arbeitselektrode ist, die auf Vorhandensein von Cholesterin anspricht.

3. Bestimmung von freiem oder Gesamtcholesterin, wobei das Cholesterin unter Verwendung eines Sensors nach Anspruch 1 amperometrisch quantifiziert wird.

FIG.1.

FIG.2.

FIG.3.

FIG.4.

FIG.5.

FIG.6.